# EUROPEAN PATENT APPLICATION

(11) **EP 3 689 254 A1**
(43) Date of publication of application: **05.08.2020**
(21) Application number: 18861095.0
(22) Date of filing: 01.10.2018
(51) Int. Cl.: A61B 17/02

(54) **PULL CLIP FOR ENDOSCOPE**

(30) Priority: 29.09.2017 JP 2017191971
(71) Applicant: Zeon Corporation, Tokyo 100-8246 (JP)
(72) Inventor: YAMADA, Tomohiro, Tokyo 100-8246 (JP); OBONAI, Toshifumi, Tokyo 100-8246 (JP); SAKAKI, Kohei, Tokyo 100-8246 (JP)
(74) Representative: Ehlich, Eva Susanne
(86) International application number: PCT/JP2018/036682
(87) International publication number: WO 2019/066084

(57) **Abstract**

[Object] To reduce the cost required for a procedure.

[Solving Means] The invention includes: a clip main body 2 including a ring 24 externally fitted in a slidable manner to a joining portion 21 on the proximal end sides of a pair of arm portions spreading out in a substantially V shape; and a loop member 4 made of a thermoplastic polymer attached to a part of the clip main body via a spring 3. The loop member includes a loop portion 41 formed in a loop shape and has, at a part of the loop portion, a fragile portion cuttable with a force weaker than at the other part. By using a clip device 5 having a sheath 52 and an engagement member (coupling hook 51) disposed so as to be capable of advancing from and retracting into the sheath 52 and detachably engaging with the joining portion 21, it is possible to cut the loop portion 41 by means of the force that is generated by the distal end of the sheath and the engagement member by pulling the engagement member from the distal end of the sheath in a state where the engagement member is engaged with a part of the loop portion.

## Description

### TECHNICAL FIELD

The present invention relates to a traction clip for an endoscope inserted into a body by means of an endoscope and used for a part of body tissue to be pulled.

### BACKGROUND ART

Performed in some cases of endoscopic submucosal dissection (ESD) is a procedure of incising a lesion site as a whole while lifting the lesion site after peeling with a traction force applied to the lesion site in advance. The procedure, which is to excise the lesion site with ease, more reliability, and so on, is performed such that the peeled lesion site does not hinder continuous incision when the lesion site is partially incised and peeled. Known as clips for an endoscope used in such procedures are, for example, the traction clips that are described in Patent Document 1 and Patent Document 2 (lifting and spring-attached clips).

The lifting clip described in Patent Document 1 is generally configured to include a clip having a pair of gripping claws (claws), an elastic member made of string-shaped rubber or the like, and a ring-shaped member (engagement member). One end of the elastic member is connected to the root part of the clip via a string-shaped connecting member and the ring-shaped member is attached to the other end of the elastic member.

When a lesion site is excised, a peeled part of the lesion site is gripped by the traction clip and the ring-shaped member is scooped up between a pair of claws of another, separately prepared, clip (normal clip lacking an elastic member, a ring-shaped member, or the like). Then, a part of a mucous membrane (such as a part of the mucous membrane facing the lesion site) is gripped by the separately prepared clip while the elastic member is elastically deformed (stretched). As a result, a traction force acts on the lesion site by the elastic force (tension) of the elastic member, and thus the lesion site peeled as a result of incision is lifted. Accordingly, a surgical field can be ensured without the field of view being blocked by the peeled lesion site, and thus the entire circumference of the lesion site can be incised with ease and reliability.

Although no specific details of the ring-shaped member are described in Patent Document 1, it is described in Patent Document 1 that a material and thinness allowing easy cutting by means of a loop cutter or the like are desirable for post-incision lesion site recovery. In addition, it is described in Patent Document 2 that a surgical suture thread can be used as a string member corresponding to the ring-shaped member.

By the way, in the related art, the ring-shaped member is cut by a cutting instrument such as a high-frequency knife and endoscopic scissors forceps also called a loop cutter being inserted via an endoscopic treatment instrument guide tube. However, it is cumbersome to separately prepare such a cutting instrument, the separation preparation leads to an increase in the cost required for a procedure, and thus the separation preparation is not preferable.

### CITATION LIST

### PATENT DOCUMENT

Patent Document 1: JP 2008-62004 A
Patent Document 2: JP 2015-192726 A

### SUMMARY OF THE INVENTION

### PROBLEM TO BE SOLVED BY THE INVENTION

The invention has been made in view of such points, and an object of the invention is to provide a traction clip for an endoscope with which no cutting instrument needs to be separately prepared and the cost required for a procedure can be reduced.

### MEANS FOR SOLVING PROBLEM

A traction clip for an endoscope according to a first aspect of the invention includes a clip main body including a pair of arm portions spreading out in a substantially V shape and a ring-shaped tightening ring externally fitted in a slidable manner to a joining portion on proximal end sides of the arm portions and a loop member made of a thermoplastic polymer attached to a part of the clip main body directly or via a connecting member, the loop member including a loop portion formed in a loop shape and having, at a part of the loop portion, a fragile portion cuttable with a force weaker than at the other part.

The traction clip for an endoscope according to the first aspect of the invention has, at a part of the loop portion, the fragile portion cuttable with a force weaker than at the other part. Accordingly, the loop portion can be easily cut with a weaker force at the part of the fragile portion by means of a clip device (described later) or the like.

In the traction clip for an endoscope according to the first aspect of the invention, a part of the loop portion is capable of serving as the fragile portion by being formed thinner than the other part.

A traction clip for an endoscope according to a second aspect of the invention includes a clip main body including a pair of arm portions spreading out in a substantially V shape toward a distal end sides and a ring-shaped tightening ring externally fitted in a slidable manner to a joining portion on proximal end sides of the arm portions, the tightening ring being pushed and slid by a distal end of a sheath and closing the arm portions by an engagement member being pulled from the distal end of the sheath in a state where the engagement member is engaged with the joining portion by a clip device being used, the clip device having the sheath and the engagement member, and the engagement member being disposed so as to be capable of advancing from and retracting into the sheath and detachably engaging with the joining portion and a loop member made of a thermoplastic polymer attached to a part of the clip main body directly or via a connecting member, the loop member including a loop portion formed in a loop shape, the engagement member of the clip device being engageable with a part of the loop portion, and the loop member being configured to be cut by the engagement member being pulled from the distal end of the sheath in a state where the engagement member is engaged with a part of the loop portion by the clip device being used.

With the traction clip for an endoscope according to the second aspect of the invention, a part of the loop member can be cut by means of the clip device. Accordingly, there is no need to prepare a separate cutting instrument for cutting the loop member and it is possible to reduce the cost required for a procedure.

A traction clip for an endoscope according to a third aspect of the invention includes a clip main body including a pair of arm portions spreading out in a substantially V shape toward a distal end side and a ring-shaped tightening ring externally fitted in a slidable manner to a joining portion on proximal end sides of the arm portions, the tightening ring being pushed and slid by a distal end of an inner sheath and closing the arm portions by an engagement member being pulled from the distal end of the inner sheath in a state where the engagement member is engaged with the joining portion by a clip device being used and the clip device having the inner sheath, the engagement member disposed so as to be capable of advancing from and retracting into the inner sheath and detachably engaging with the joining portion, and an outer sheath through which the inner sheath is slidably inserted and a loop member made of a thermoplastic polymer attached to a part of the clip main body directly or via a connecting member, the loop member including a loop portion formed in a loop shape, the engagement member of the clip device being engageable with a part of the loop portion, and the loop member being configured to be cut by a cutting clip being pulled from a distal end of the outer sheath in a state where the engagement member is engaged with a joining portion of a cutting clip substantially similar configuration with the clip main body, the cutting clip is connected to the distal end of the inner sheath, and a part of the loop portion is gripped by a pair of arm portions of the cutting clip being closed, while using the clip device.

With the traction clip for an endoscope according to the third aspect of the invention, a part of the loop member can be cut by means of the clip device to which the cutting clip is connected. Accordingly, there is no need to prepare a separate cutting instrument for cutting the loop member and it is possible to reduce the cost required for a procedure. In addition, the cutting clip can be easily replaced after cutting, and thus a new cutting clip can be used each time cutting is repeated by means of the same clip device.

Usable as the clip device in the traction clip for an endoscope according to the second and third aspects of the invention is a clip device in which the engagement member has a coupling hook spreading out in a substantially V shape by being pushed out from the distal end of the sheath and closing by being pulled into the sheath (or the inner sheath). When the clip device is used, the loop member can be gripped by the coupling hook being closed and the loop member and the coupling hook can be engaged with each other as a result. Accordingly, an engagement operation (work) is facilitated and the loop member can be cut with ease.

### BRIEF DESCRIPTION OF DRAWINGS

Fig. 1A is a diagram illustrating an overall configuration in a state where arm portions of a clip main body of a traction clip for an endoscope of an embodiment of the invention are spread out;
Fig. 1B is a diagram in which the clip main body constituting the traction clip in Fig. 1A is changed in direction;
Fig. 1C is a diagram illustrating a state where the arm portions of the clip main body of the traction clip in Fig. 1A are closed;
Fig. 2A is a diagram illustrating a coil spring of the traction clip in Fig. 1A;
Fig. 2B is a diagram in which the coil spring in Fig. 2A is viewed from a loop member mounting side;
Fig. 3A is a plan view of a loop member of the traction clip in Fig. 1A;
Fig. 3B is a front view of the loop member in Fig. 3A;
Fig. 4A is a plan view illustrating a modification example of the loop member in Fig. 3A;
Fig. 4B is a plan view illustrating another modification example of the loop member in Fig. 3A;
Fig. 4C is a plan view illustrating yet another modification example of the loop member in Fig. 3A;
Fig. 4D is a front view of the loop member in Fig. 4C;
Fig. 5A is a diagram illustrating the appearance of a clip device of the embodiment of the invention;
Fig. 5B is a cross-sectional view taken along line Vb-Vb in Fig. 5A;
Fig. 5C is a perspective view illustrating the configuration of a coupling hook of the clip device in Fig. 5A and the vicinity of the coupling hook;
Fig. 6A is a diagram illustrating a state where the traction clip in Fig. 1A protrudes from the distal end of the clip device;
Fig. 6B is a diagram illustrating a state where the traction clip in Fig. 1A is stored at the distal end of the clip device;
Fig. 7A is a diagram schematically illustrating a state where a part of a lesion site is in the process of incision by means of the traction clip in Fig. 1A;
Fig. 7B is a diagram schematically illustrating a state where the distal end of the clip device is brought close for the loop member to be cut after the incision of the lesion site in Fig. 7A is completed;
Fig. 8A is a diagram illustrating a process of cutting the loop member of the traction clip in Fig. 1A and is a diagram illustrating a state where the coupling hook protrudes with the distal end of the clip device disposed in the vicinity of the loop member;
Fig. 8B is a diagram illustrating a process subsequent to Fig. 8A and is a diagram illustrating a state where a part of the loop member is gripped by the coupling hook, the coupling hook is pulled from the distal end of a sheath, and the loop member abuts against the distal end of an inner sheath;
Fig. 8C is a diagram illustrating a process subsequent to Fig. 8B and is a diagram illustrating a state where the coupling hook is further pulled from the distal end of the inner sheath and the loop member is cut by the force that is generated in the loop member by the distal end of the inner sheath and the coupling hook;
Fig. 9A is a plan view illustrating another modification example of the loop member in Fig. 3A;
Fig. 9B is a front view of the loop member in Fig. 9A;
Fig. 9C is a side view of the loop member in Fig. 9A;
Fig. 9D is a cross-sectional view taken along line IXd-IXd in Fig. 9A;
Fig. 9E is a cross-sectional view taken along line IXe-IXe in Fig. 9A;
Fig. 10A is a diagram illustrating another process of cutting the loop member of the traction clip in Fig. 1A and is a diagram illustrating a state where a cutting clip connected to the clip device is disposed in the vicinity of the loop member;
Fig. 10B is a diagram illustrating a process subsequent to Fig. 10A and is a diagram illustrating a state where a tightening ring is slid and a part of the loop member is gripped by the cutting clip; and
Fig. 10C is a diagram illustrating a process subsequent to Fig. 10B and is a diagram illustrating a state where the loop member is cut by the force that is generated in the loop member by the distal end of an outer sheath and the cutting clip by the cutting clip being pulled from the distal end of the outer sheath with the distal end of the outer sheath abutting against the loop member.

### MODE(S) FOR CARRYING OUT THE INVENTION

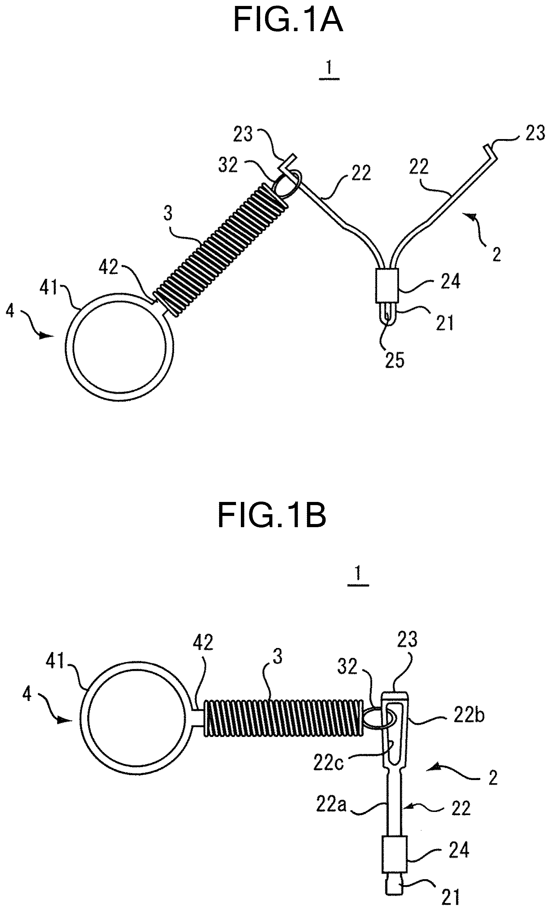

Hereinafter, an embodiment of the invention will be described in detail with reference to the drawings. As illustrated in Figs. 1A and 1B, a traction clip 1 for an endoscope according to the present embodiment is generally configured to include a clip main body 2, a coil spring 3, and a loop member 4 and the traction clip 1 for an endoscope according to the present embodiment is a spring-attached clip using the coil spring 3 as a connecting member interconnecting the clip main body 2 and the loop member 4.

However, the coil spring 3 is an example of the connecting member and rubber, another elastic member, or a non-elastic member may also be used as the connecting member. In addition, the connecting member may be omitted and the loop member 4 may be directly connected (attached) to the clip main body 2 not via the connecting member.

The clip main body 2 includes a joining plate portion (joining portion) 21, a pair of arm plate portions (arm portions) 22, and a tightening ring 24. The joining plate portion 21 has a shape bent in a substantially U shape and the arm plate portions 22 are integrally formed so as to be respectively continuous with the end portions of the U shape of the joining plate portion 21 and spread out in a substantially V shape toward the distal end side of the joining plate portion 21.

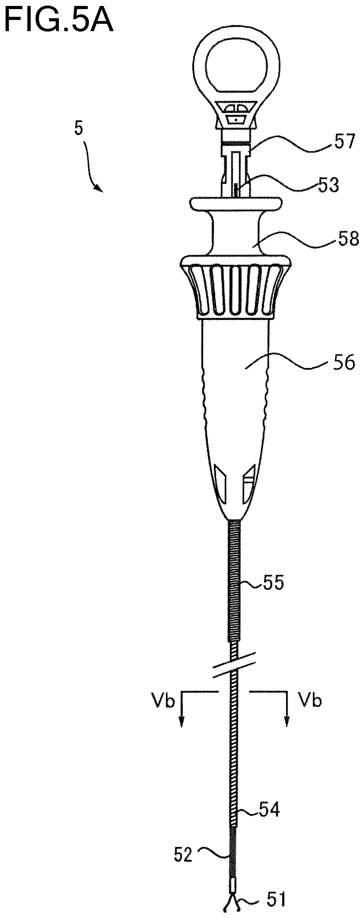

The tightening ring 24 is a member formed in a ring shape and is externally fitted in a slidable manner to the joining plate portion 21 on the proximal end side of the arm plate portion 22. The tightening ring 24 is a member that is slid by means of a clip device (see Figs. 5A to 5C, described in detail later), the clip device includes an inner sheath (sheath) 52 and a coupling hook (engagement member) 51, and the coupling hook (engagement member) 51 is disposed so as to be capable of advancing from and retracting into the inner sheath 52 and detachably engages with (is detachably connected to) the joining plate portion 21. The tightening ring 24 is pushed and slid by the distal end of the inner sheath 52 and closes the arm plate portions 22 by the coupling hook 51 being pulled from the distal end of the inner sheath 52 in a state where the coupling hook 51 is connected to the joining plate portion 21.

Claw portions 23 are integrally formed in the respective distal ends of the arm plate portions 22. The claw portion 23 is formed by being bent toward the inside (that is, the closing direction) at the distal end of the arm plate portion 22. Each claw portion 23 includes a notch portion (not illustrated) recessed at the intermediate part of the distal end.

The joining plate portion 21, the pair of arm plate portions 22, and the pair of claw portions 23 constituting the clip main body 2 are formed by a thin and elongated plate material being bent. Although not particularly limited, the plate thickness of the plate material constituting the clip main body 2 is preferably 0.10 to 0.30 mm. An elastic plate material is preferable as the plate material and stainless steel or the like is used as the plate material.

As illustrated in Fig. 1B, each of the arm plate portions 22 includes a proximal end 22a and a grip portion 22b. A penetration portion 22c is formed in the grip portion 22b of each arm plate portion 22. The penetration portions 22c are formed without impairing the desired strength of the arm plate portions 22 (grip portions 22b). One of the penetration portions 22c is also used for the coil spring 3 to be connected to the clip main body 2 as will be described in detail later.

The tightening ring 24 is slidably fitted to the joining plate portion 21. A substantially cylindrical ring member constitutes the tightening ring 24. However, the tightening ring 24 may include a spring formed by a wire rod being wound in a coil shape. The joining plate portion 21 is inserted through the guide hole that is inside the tightening ring 24 and the tightening ring 24 is mounted (externally fitted) so as to be axially movable (slidable) between the outer periphery of the joining plate portion 21 and the outer periphery of the proximal end 22a of the arm plate portion 22.

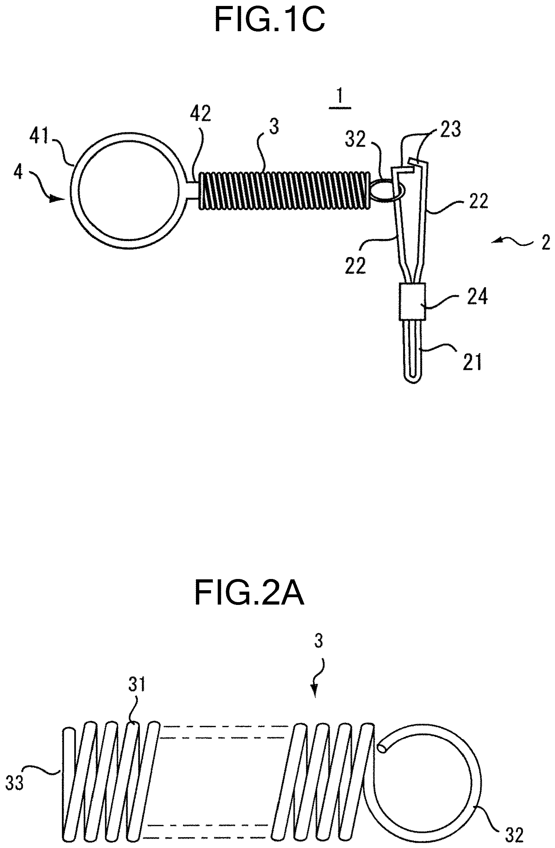

As illustrated in Fig. 1A or 1B, in a state where the tightening ring 24 is disposed close to the back (joining plate portion 21), the arm plate portions 22 are open (spread out) by the elasticity of the arm plate portions 22. If necessary, the arm plate portions 22 can be closed by the tightening ring 24 being moved (slid) to a position close to the distal end (the proximal end 22a) as illustrated in Fig. 1C.

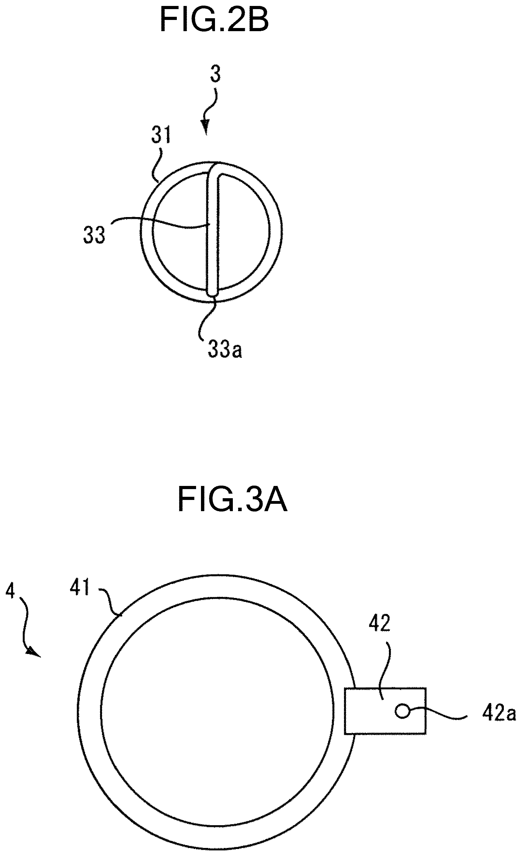

The coil spring 3 is a tension coil spring formed by a metal wire such as a stainless steel wire being wound. As illustrated in Figs. 2A and 2B, the coil spring 3 includes a clip hook 32 for connection to the clip main body 2 at one end of a main body portion 31 formed by a metal wire being wound and a linear loop member hook 33 for mounting (attaching) the loop member 4 at the other end of the main body portion 31. The clip hook 32 is formed by a part corresponding to approximately one lap (or at least one lap) at one end of the substantially cylindrically wound metal wire (main body portion 31) being bent by approximately 90 degrees with respect to a surface orthogonal to the axis of the main body portion 31. It should be noted that the clip hook 32 may be separately prepared and welded and fixed to one end of the wound metal wire.

The loop member hook 33 is formed by a part of the other end of the main body portion 31 being bent inward so as to draw a substantially D shape and linearly molded. It is preferable from the viewpoint of preventing the loop member 4 from escaping that a distal end 33a of the loop member hook 33 that is linearly molded reaches the wound metal wire (main body portion 31) as illustrated in Fig. 2B when the loop member 4 (described later) is connected. However, in a case where the loop member 4 is connected and then fixed by adhesion or the like, the distal end of the linear portion that is linearly molded does not have to reach the wound metal wire (main body portion 31).

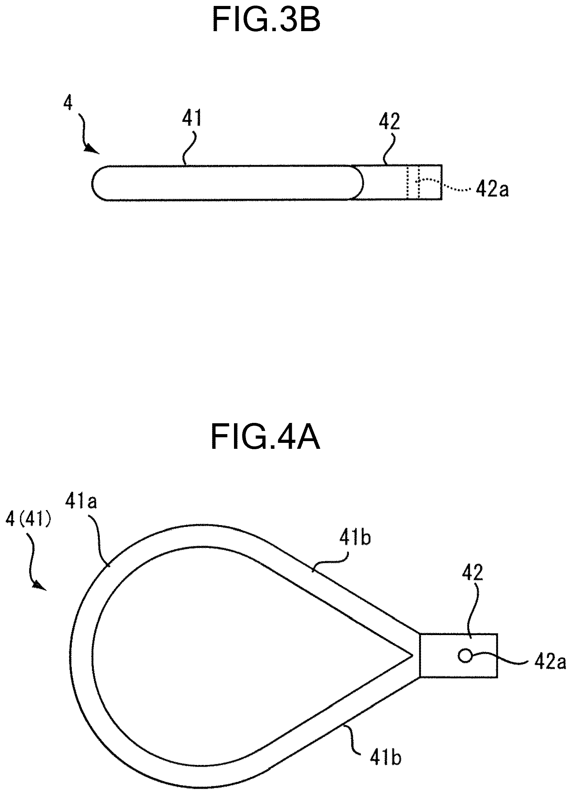

As illustrated in Figs. 3A and 3B, the loop member 4 is made of a thermoplastic polymer material molded component attached to a part of the clip main body 2 via the coil spring (connecting member) 3 (injection molded product of a thermoplastic polymer in the present embodiment), the loop member 4 includes an adapter portion 42 for connection to the coil spring 3 and a loop portion 41 formed in a loop shape (so as to be endless), and the coupling hook (engagement member) 51 of a clip device 5 can be connected to (engaged with) a part of the loop portion 41. In the present embodiment, the shape of the loop portion 41 is a substantially annular shape in a plan view having a uniform cross-sectional shape (substantially circular shape) over the entire circumference.

The loop portion 41 is flexible enough to be stored in the distal end of an outer sheath 54 of the clip device 5 (described later) and is configured to be cut by the shearing force that is generated by the distal end of the inner sheath 52 and the coupling hook 51 by the coupling hook 51 being pulled from the distal end of the inner sheath 52 in a state where the coupling hook (engagement member) 51 is connected to (engaged with) a part of the loop portion 41 by means of the clip device 5.

More specifically, the loop portion 41 is set in terms of, for example, shape (loop shape and cross-sectional shape), dimension (loop diameter and cross-sectional diameter), material, molding method, and molding condition (molding temperature, pressure, and so on) in relation to the inner diameter of the distal end of the outer sheath 54, the inner diameter of the distal end of the inner sheath 52, the configuration of the coupling hook 51, and the like so as to have both the flexibility that allows the loop portion 41 to be stored in the outer sheath 54 and the brittleness that allows the loop portion 41 to be cut by the shearing force generated by the distal end of the inner sheath 52 and the coupling hook 51.

In the present embodiment, the adapter portion 42 is a substantially rectangular plate-shaped part formed integrally with the loop portion 41 and has a spring through hole 42a penetrating the adapter portion 42 in a direction substantially perpendicular to a plate surface. The spring through hole 42a is a substantially circular hole allowing the loop member hook (linear portion) 33 of the coil spring 3 to be fitted. The inner diameter of the spring through hole 42a is set to a value equal to or slightly larger than the outer diameter of the loop member hook (linear portion) 33 of the coil spring 3.

Although the adapter portion 42 is formed by being injection-molded integrally with the loop portion 41 in the present embodiment, the adapter portion 42 may be an adapter member independent of the loop portion and may be joined to the loop portion 41. In this case, the adapter member is not limited to a substantially rectangular plate-shaped adapter member such as the adapter portion 42 and an adapter member having another shape and another configuration may be used insofar as the function of interconnecting the coil spring 3 and the loop portion 41 can be realized.

As for the connection of the loop member 4 to the coil spring 3, the open end side of the adapter portion 42 is inserted and disposed so as to be positioned inside the coil spring 3 (main body portion 31) and the elastic deformation of the adapter hook 33 is released in a state where the loop member hook (linear portion) 33 of the coil spring 3 is fitted (inserted) through the spring through hole 42a of the adapter portion 42 while the loop member hook (linear portion) 33 of the coil spring 3 is elastically deformed to the outside (direction in which the distal end 33a is separated from the end of the main body portion 31) as necessary. As a result, the loop member 4 is connected (attached) to the coil spring 3. In this manner, the adapter portion 42 is easily connected to the coil spring 3 and, in addition, the adapter portion 42 is prevented from falling from the coil spring 3. It should be noted that adhesion may be performed on the contact part between the loop member hook 33 and the spring through hole 42a of the adapter portion 42b so that the adapter portion 42 is more reliably prevented from falling from the coil spring 3.

A thermoplastic polymer is used as the material of the loop member 4. Specific examples of the material include a thermoplastic resin such as polypropylene, polyethylene, polyamide, and polystyrene and a thermoplastic elastomer such as a polyolefin-based elastomer, a polyamide-based elastomer, a polystyrene-based elastomer, and a polyurethane-based elastomer. The diameter (outside diameter) of the loop portion 41 can be approximately ϕ2 to 6 mm and the cross-sectional diameter of the loop portion 41 can be approximately 0.2 to 0.5 mm. The diameter of the spring through hole 42a can be approximately 0.1 to 0.3 mm.

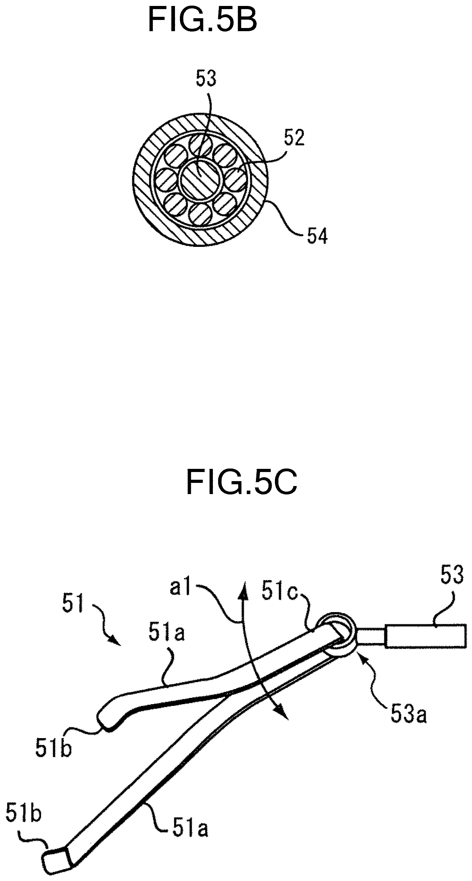

The traction clip 1 for an endoscope as described above is mounted and stored in the distal end of the clip device 5 and the sheath portion of the clip device 5 is inserted into the lumen of an endoscope and guided to biological tissue as a procedure target. Here, an example of the clip device will be described with reference to Figs. 5A and 5B.

The clip device 5 is generally configured to include the coupling hook 51, the inner sheath 52, a drive wire 53, the outer sheath 54, a reinforcing coil 55, a slider 56, a base portion 57, and an operation portion 58.

The similarly tubular inner sheath 52 is inserted through the tubular outer sheath 54 and the drive wire 53 is inserted through the inner sheath 52. The inner sheath 52 is slidable in the outer sheath 54 and the drive wire 53 is slidable in the inner sheath 52.

The outer sheath 54 is made of a flexible hollow tube and a coil tube is used for the outer sheath 54 in the present embodiment. Usable as the coil tube is a flat wire coil tube formed by a long flat plate made of metal (stainless steel) or the like being spirally wound. However, a round wire coil tube or an inner flat coil tube may also be used. The inner diameter of the distal end of the outer sheath 54 is approximately 2 to 3 mm.

The inner sheath 52 is made of a flexible hollow tube and a wire tube is used for the inner sheath 52 in the present embodiment. The wire tube is a tube made of a hollow stranded wire formed by a plurality of wires (cables) made of metal (stainless steel) or the like being spirally twisted so as to become hollow. It should be noted that the wire tube may be mainly used for the inner sheath 52 with a coil tube used for only a part of the distal end side of the inner sheath 52. The inner diameter of the distal end of the inner sheath 52 is approximately 1.5 to 2.5 mm.

The drive wire 53 is made of a flexible wire and a wire rope is used for the drive wire 53 in the present embodiment. The wire rope is a rope made of a stranded wire formed by a plurality of wires (cables) made of metal (stainless steel) or the like being spirally twisted. However, a wire tube similar to the inner sheath 52 may also be used as the drive wire 53.

As illustrated in Fig. 5C, the coupling hook 51 disposed at the sheath distal end of the clip device 5 includes a pair of arm portions 51a and 51a made of elastic bodies disposed in a substantially V shape toward the distal end of the coupling hook 51 and is capable of having two states in cooperation with the inner sheath 52, one being a spread-out (open) state and the other being a closed state. Claw portions 51b and 51b are formed in the distal ends of the arm portions 51a and 51a of the coupling hook 51 by being bent to the inside (side of being relative to each other) and the joining plate portion 21 of the clip main body 2 can be gripped and connected.

The proximal end of the coupling hook 51 is a U-shaped portion 51c formed in a substantially U shape continuously with the proximal ends of the pair of arm portions 51a and 51a. The U-shaped portion 51c and the arm portions 51a and 51a including the claw portions 51b and 51b can be formed by an elongated plate material made of an elastic body being appropriately bent (plastically deformed). Although not particularly limited, the plate thickness of the plate material constituting the coupling hook 51 is approximately 0.20 to 0.24 mm and the width of the plate material constituting the coupling hook 51 is approximately 0.6 mm. Stainless steel or the like is used as the plate material.

An annular member 53a formed in a substantially annular shape is integrally welded and fixed by laser welding or the like to the distal end of the drive wire 53 slidably inserted in the inner sheath 52. The annular member 53a has an outer diameter of approximately 1.2 mm and an inner diameter of approximately 0.8 to 1.0 mm.

The coupling hook 51 is inserted through the annular member 53a and is disposed such that the U-shaped portion 51c is loosely fitted to the annular member 53a. As a result, the coupling hook 51 is swingably attached to the distal end of the drive wire 53. By the coupling hook 51 being attached to the distal end of the drive wire 53 via the annular member 53a and the U-shaped portion 51c loosely fitted to the annular member 53a, the coupling hook 51 is capable of freely pivoting in the arrow a1 direction in Fig. 5C.

Returning to Figs. 5A and 5B, the vicinity of the proximal end side of the outer sheath 54 is inserted in the reinforcing coil 55 and integrally fixed to the reinforcing coil 55. The reinforcing coil 55 is integrally fixed to the slider 56 and the distal end side part of the base portion 57 is inserted and disposed inside the slider 56. The slider 56 is slidable with respect to the base portion 57 such that the slider 56 can be positioned between a position of movement to the distal end side and two positions of movement to the proximal end (proximal end) side.

The operation portion 58 is slidably held on the base portion 57 and the inner sheath 52 is fixed to the base portion 57. The proximal end of the drive wire 53 is fixed to the operation portion 58.

When the operation portion 58 is slid to the distal end side with respect to the base portion 57, the inner sheath 52 is pulled with respect to the drive wire 53 and the coupling hook 51 at the distal end of the drive wire 53 protrudes from the distal end of the inner sheath 52 and spreads out by the elasticity of the coupling hook 51. When the operation portion 58 is slid to the proximal end side with respect to the base portion 57, the drive wire 53 is pulled with respect to the inner sheath 52 and the coupling hook 51 at the distal end of the drive wire 53 is gradually closed while entering the inner sheath 52 and completely closed by being embedded into the inner sheath 52.

When the slider 56 is slid to the proximal end side position with respect to the base portion 57, the inner sheath 52 is capable of protruding from the distal end of the outer sheath 54. In contrast, when the slider 56 is slid to the distal end side position with respect to the base portion 57, the distal end of the inner sheath 52 can be stored (embedded) in the outer sheath 54.

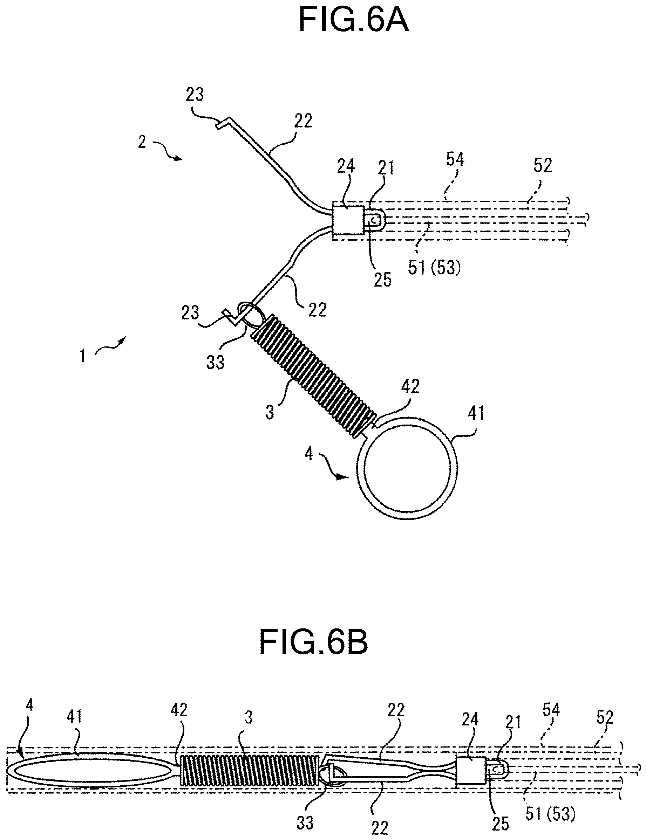

Next, a method for using the traction clip 1 for an endoscope described above will be described. In the clip main body 2, a connecting hole 25 is formed in the joining plate portion 21 having a U-shaped cross section (see Figs. 1A and 6A) in a state where the tightening ring 24 is externally fitted close to the proximal end 22a on the outer periphery of the joining plate portion 21. The coupling hook 51 in Fig. 5A is engaged with the connecting hole 25 and the coupling hook 51 is pulled into the inner sheath 52, and then the coupling hook 51 is closed and the clip main body 2 of the traction clip 1 for an endoscope is attached to the distal end of the inner sheath 52 (see Fig. 6A).

In this state, the distal end of the inner sheath 52 to which the traction clip 1 for an endoscope (clip main body 2, coil spring 3, and loop member 4) is connected is pulled into the outer sheath 54. Then, the entire traction clip 1 for an endoscope (clip main body 2, coil spring 3, and loop member 4) is stored inside the distal end of the outer sheath 54 as illustrated in Fig. 6B. In this state, the tightening ring 24 of the clip main body 2 remains positioned on the joining plate portion 21 and the arm plate portion 22 is closed by the action of the inner wall of the outer sheath 54. In addition, the loop portion 41 of the loop member 4 is stored in the outer sheath 54 in an elastically deformed state by the action of the inner wall of the outer sheath 54.

By means of an endoscope (not illustrated) and with the traction clip 1 for an endoscope mounted, the distal end of the sheath portion of the clip device 5 is positioned in the vicinity of biological tissue as an incision (peeling) procedure target. Next, the traction clip 1 for an endoscope is allowed to protrude from the distal end of the outer sheath 54 by the outer sheath 54 being slid to the proximal end side. As a result, the arm plate portion 22 spreads out by the elasticity of the arm plate portion 22 and the loop portion 41 of the loop member 4 returns to the original shape of the loop portion 41 by the elasticity of the loop portion 41 as illustrated in Fig. 6A.

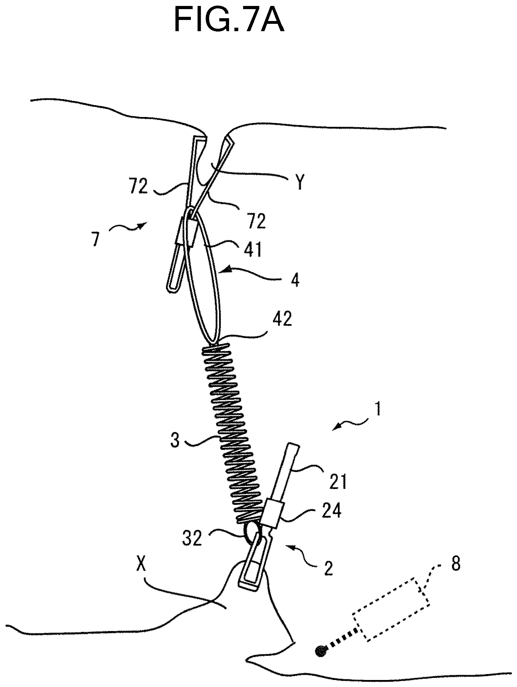

With the arm plate portion 22 spread out, the arm plate portion 22 is positioned in the vicinity of an excision (peeling) target site (lesion site) X of the mucous membrane (biological tissue) as illustrated in, for example, Fig. 7A. Next, the tightening ring 24 is slid to the distal end side of the arm plate portion 22 by the inner sheath 52 being slid to the distal end side with respect to the drive wire 53. As a result, the arm plate portions 22 are gradually closed (approach each other) and the mucous membrane on one side and the mucous membrane on the other side are pulled together across the lesion site X.

By the inner sheath 52 being further slid to the distal end side with respect to the drive wire 53, the tightening ring 24 moves to the grip portion 22b side of the arm plate portion 22 (see Fig. 1C) and the lesion site X is completely gripped by the traction clip 1 for an endoscope (clip main body 2). In this state, the inner sheath 52 is slid to the proximal end side with respect to the drive wire 53. Then, the coupling hook 51 is pushed out from the distal end of the inner sheath 52 and spreads out, the gripping (engagement) of the traction clip 1 for an endoscope (clip main body 2) by the coupling hook 51 is released, and the lesion site X is completely clipped by the traction clip 1 for an endoscope (clip main body 2).

Next, the clip device 5 is temporarily removed from the endoscope. Then, a separately prepared clip (normal clip) 7 is attached to the distal end of the clip device 5 (or a separately prepared clip device similar configuration with the clip device 5) and the distal end of the sheath portion of the clip device 5 to which the normal clip 7 is attached is transported to the vicinity of an appropriate part (facing part) Y facing (opposite to) the lesion site X. It should be noted that a clip similar configuration with the clip main body 2 in which the coil spring 3 and the loop member 4 are removed from the traction clip 1 for an endoscope illustrated in Figs. 1A to 1C can be used as the normal clip 7.

Next, the loop portion 41 of the loop member 4 is scooped up by one of the pair of arm plate portions of the normal clip 7 (a part of the loop portion 41 is positioned at the part between the pair of arm plate portions) and the normal clip 7 is allowed to clip the facing part Y as in the case of the above-described gripping of the lesion site X by the clip main body 2 while the coil spring 3 is extended. As a result, the lesion site X is pulled (lifted) by the elastic force (tension) of the coil spring 3. Subsequently, an electric knife 8 for an endoscope is inserted via the endoscope and the outer periphery of the lesion site X is incised by means of the electric knife 8. The lesion site X is lifted by the elastic force (tension) of the coil spring 3 from the part of peeling resulting from the incision, and thus the entire circumference of the lesion site X can be easily and reliably incised without the part of peeling that has resulted from the incision of the lesion site X blocking the field of view or hindering the procedure.

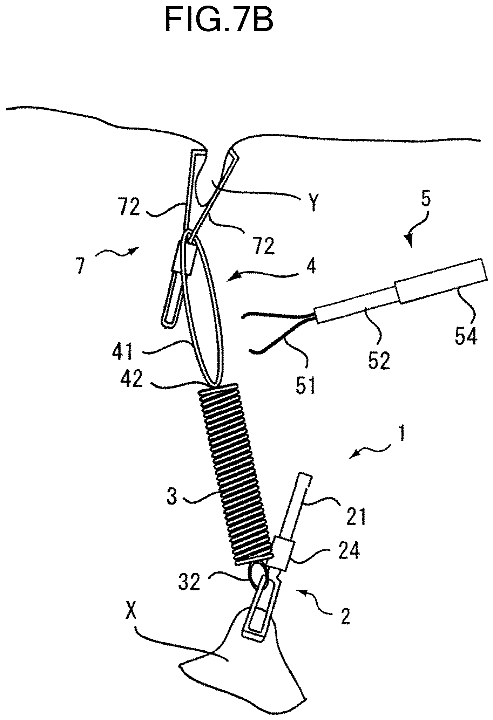

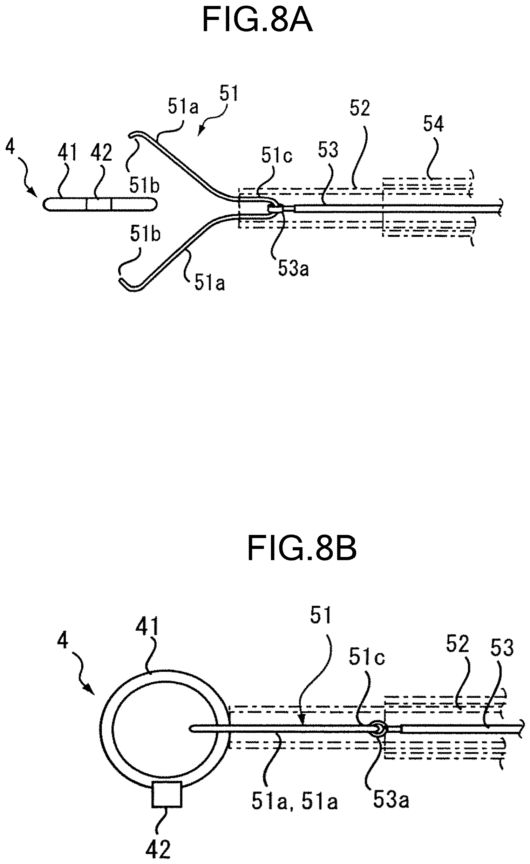

Next, as illustrated in Fig. 7B, the electric knife 8 for an endoscope is removed from the endoscope with the lesion site X completely excised, the distal end of the clip device 5 (or the separately prepared clip device similar configuration with the clip device 5) to which the clip is not attached (connected) is positioned in the vicinity of the loop portion 41 of the loop member 4 that is in the process of traction, and the coupling hook 51 is allowed to spread out by protruding from the distal end of the inner sheath 52. As illustrated in Fig. 8A, in this state, the position of the loop portion 41 is finely adjusted such that an appropriate part of the loop portion 41 can be gripped.

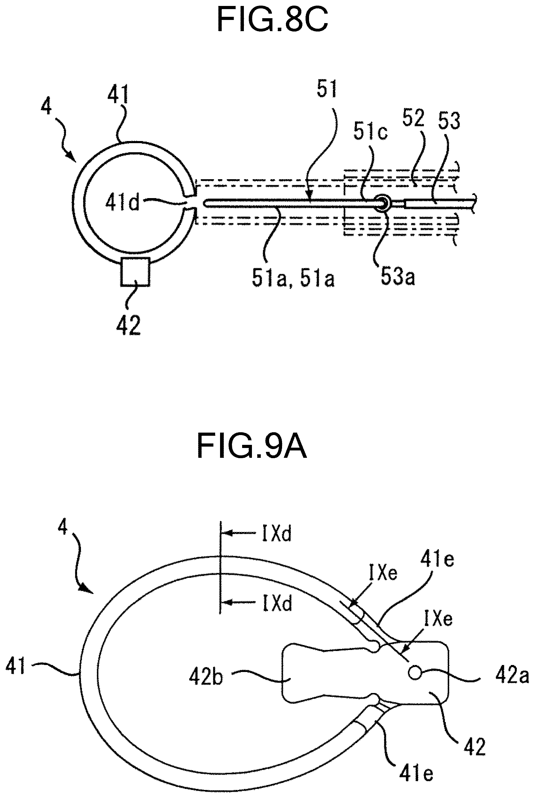

Next, the inner sheath 52 is pushed out to the distal end side with respect to the drive wire 53 and the coupling hook 51 is closed. Then, an appropriate part of the loop portion 41 is gripped by the coupling hook 51 and the loop portion 41 is allowed to abut against the distal end of the inner sheath 52 as illustrated in Fig. 8B. Subsequently, the drive wire 53 is slid to the proximal end side and pulled with respect to the inner sheath 52 and the appropriate part of the loop portion 41 is cut by the shearing force generated by the distal end of the inner sheath 52 and the coupling hook 51 as illustrated in Fig. 8C. It should be noted that the lesion site X can be gripped by the coupling hook 51 of the clip device 5, gripping forceps for an endoscope, or the like together with the traction clip 1 and extracted from the body after the loop portion 41 is cut.

The loop member 4 (loop portion 41) of the traction clip 1 for an endoscope according to the embodiment described above can be cut by means of the clip device 5 sliding the tightening ring 24 so that the pair of arm plate portions 22 and 22 of the clip main body 2 are closed, and thus there is no need to separately prepare a cutting instrument for cutting the loop member 4 and it is possible to reduce the cost required for a procedure. In addition, the loop member 4 of the traction clip 1 for an endoscope according to the embodiment described above integrally includes the loop portion 41 and the adapter portion 42, and thus an adapter member as described in Patent Document 2 does not have to be separately prepared and the loop member 4 has a simple configuration.

Although the outer shape of the loop portion 41 of the loop member 4 is substantially circular in plan view in the embodiment described above, the invention is not limited thereto and the shape may be an elliptical shape, an oval shape, a polygonal shape (such as a rhombic shape), or a loop shape in which two or more of the shapes are combined after partial cutting. For example, the loop shape may include a semicircular (arc-shaped) part 41a and a semi-rhombic (V-shaped) part 41b as illustrated in Fig. 4A.

In addition, although the position of the adapter portion 42 with respect to the loop portion 41 in the present embodiment may be any position since the loop portion 41 is substantially circular, the adapter portion 42 is preferably provided on one side in the long axis direction in a case where the loop portion 41 has a flat shape such as an elliptical shape. In a case where the loop portion 41 has the loop shape including the semicircular (or semi-elliptical) part 41a and the semi-rhombic (V-shaped) part 41b, it is preferable that the adapter portion 42 is provided at the vertex part of the V-shaped part 41b.

Further, although the cross-sectional shape of the loop portion 41 is substantially circular in the present embodiment, the invention is not limited thereto and the shape may also be elliptical, oval, or polygonal.

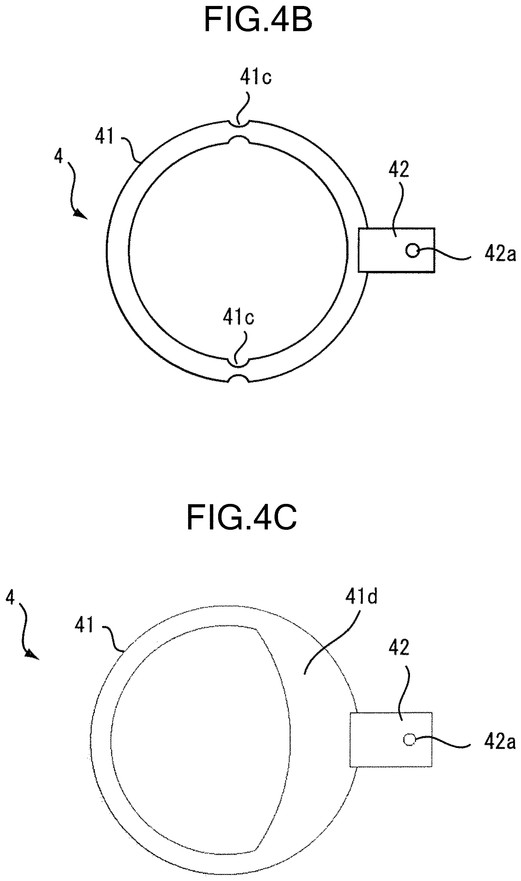

In addition, although the loop portion 41 of the loop member 4 has a uniform and substantially circular cross section over the entire circumference of the loop portion 41 in the embodiment described above, a part of the loop portion 41 of the loop member 4 may be smaller in diameter than the other part and a fragile portion (small-diameter portion 41c) may be provided such that the part can be cut with a force weaker than at the other part as illustrated in, for example, Fig. 4B. By the fragile portion being cut by means of the clip device 5, the cutting can be easily performed with a weaker force.

It should be noted that the small-diameter portion 41c as the fragile portion in the drawing is at the two positions of twelve o'clock and six o'clock with the adapter portion 42 set at the three o'clock position in view of the ease of approach by the coupling hook 51 and yet the position of the small-diameter portion 41c may be different from the positions and may be one or three or more in number. In addition, the fragile portion provided in the loop portion 41 is not limited to a small-diameter portion and the fragile portion may allow the part to be cut with a force weaker than at the other part by the internal composition of the fragile portion being changed without a change in the cross-sectional shape of the fragile portion. For example, when joining is performed by molten material inflow from both sides at the part to become the fragile portion during injection molding, the joining part (boundary part) can become fragile, and thus the fragile portion may be formed by the method.

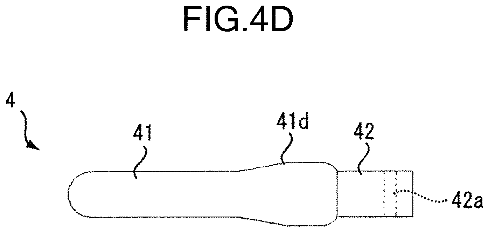

In addition, although the loop portion 41 of the loop member 4 has a uniform diameter (wire diameter) over the entire circumference of the loop portion 41 in the embodiment described above, a large-diameter portion 41d in which a part of the loop portion 41 of the loop member 4 is larger in diameter than the other part may also be provided as illustrated in, for example, Figs. 4C and 4D. By the large-diameter portion 41d being a part of the loop portion 41 and by the large-diameter portion 41d being present, it is possible to prevent the entire loop portion 41 from being pulled into the inner sheath 52 of the clip device 5 and a shearing force sufficient to cut the loop portion 41 can be applied to the loop portion 41 when the loop portion 41 (part other than the large-diameter portion 41d) is cut by means of the clip device 5.

It should be noted that the large-diameter portion 41d in the drawing is provided with a length slightly shorter than the half circumference of the loop portion 41 on the side near the adapter portion 42 in view of the ease of approach to the loop portion 41 (part other than the large-diameter portion 41d) by the coupling hook 51 and yet the large-diameter portion 41d may be formed at a different position or have a different length. In addition, although the diameter of the loop portion 41 changes in a tapered shape at the boundary between the large-diameter portion 41d and the rest of the loop portion 41, the diameter may also change in, for example, stages.

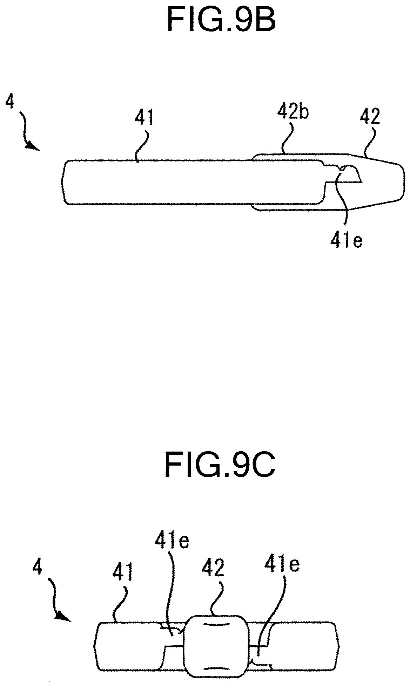

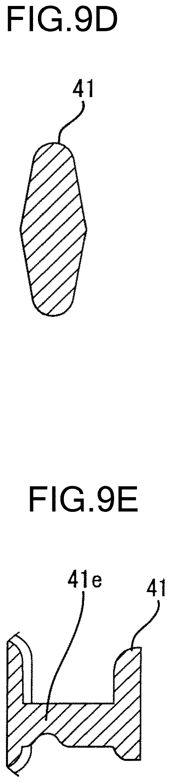

In addition, although Fig. 4B illustrates the fragile portions 41c and 41c that are provided at two locations separated from the adapter portion 42 of the loop portion 41, fragile portions 41e may be provided at two locations close to the adapter portion 42 as illustrated in Figs. 9A to 9E. By providing the fragile portions at the two locations, it is possible to select the one that is easily cut during cutting, and thus the cutting can be facilitated. However, in a case where the loop portion 41 may be insufficient in terms of strength or the arm portion of the clip through which the loop portion 41 is passed (see the normal clip 7 in Fig. 7A) is caught by the fragile portion 41c during traction or the like to result in a hindrance to the work, it is possible to reduce the problems by providing the fragile portion 41e at one location.

It should be noted that the loop portion 41 in the modification example illustrated in Figs. 9A to 9E is set in terms of, for example, shape (loop shape and cross-sectional shape), dimension (loop diameter and cross-sectional diameter), material, molding method, and molding condition (molding temperature, pressure, and so on) in relation to the inner diameter of the distal end of the inner sheath 52 and the like so as to have flexibility that allows a part of the loop portion 41 to be gripped by the coupling hook 51 of the clip device 5 and easily pulled into the inner sheath 52. In addition, in the modification example illustrated in Figs. 9A to 9E, the adapter portion 42 includes a projection portion 42b protruding to the inside of the loop portion 41. The projection portion 42b is set in terms of, for example, dimension and rigidity in relation to the inner diameter of the distal end of the inner sheath 52 and the like such that the adapter portion 42 including the projection portion 42b is caught by the distal end of the inner sheath 52 and is not easily pulled into the inner sheath 52 when a part of the loop portion 41 is gripped by the coupling hook 51 of the clip device 5 and pulled into the inner sheath 52. In the modification example illustrated in Figs. 9A to 9E, a part of the loop portion 41 is gripped by the coupling hook 51 of the clip device 5 and the drive wire 53 is pulled by being slid to the proximal end side with respect to the inner sheath 52. As a result, substantially the entire loop portion 41 is pulled into the inner sheath 52 and only the adapter portion 42 is caught by the distal end of the inner sheath 52. Then, by a force in the pulling direction being further applied to the loop portion 41, a tensile force is applied to the loop portion 41 and the loop portion 41 is cut in the fragile portion 41e.

In addition, in the embodiment described above, the loop portion 41 is gripped by the coupling hook 51 of the clip device 5 and pulled from the distal end of the inner sheath 52, and then the loop portion 41 is cut by the inner sheath 52 and the coupling hook 51 applying a cutting force to the loop portion 41. However, when the loop portion 41 is repeatedly cut by means of the clip device 5, permanent set-in fatigue (plastic deformation) may arise in the arm portion 41 of the coupling hook 51 and a sufficient gripping force required for cutting may not be exhibited.

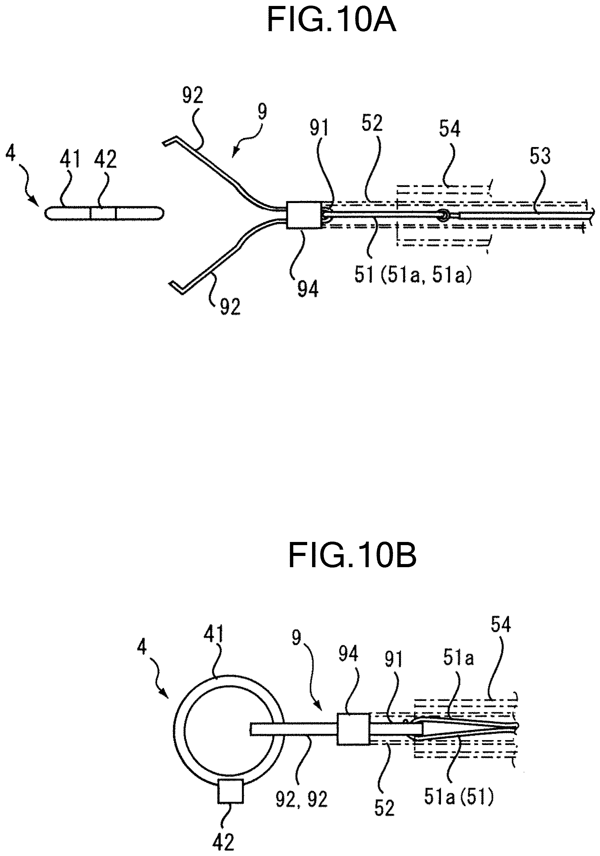

This problem may be addressed by means of the configuration that is illustrated in Figs. 10A to 10C, in which a cutting clip 9 is connected to the clip device 5, a part of the loop portion 41 is gripped by a pair of arm portions 92 and 92 (preferably the fragile portion in a case where the loop portion 41 has the fragile portion) of the cutting clip 9 being closed, and cutting is performed with the force that is generated by the cutting clip 9 and the distal end of the outer sheath 54 in that state by the cutting clip 9 being pulled from the distal end of the outer sheath 54. It should be noted that a clip (normal clip generally used as a hemostatic clip or the like) similar configuration with the clip main body 2 in which the coil spring 3 and the loop member 4 are removed from the traction clip 1 for an endoscope illustrated in Figs. 1A to 1C can be used as the cutting clip 9.

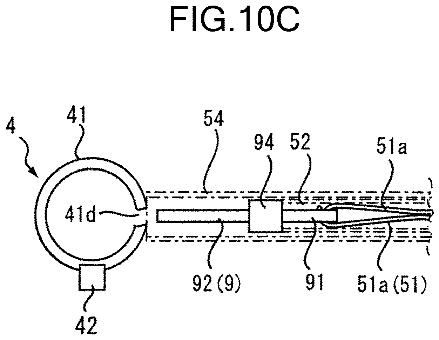

In other words, as illustrated in Fig. 10A, the position of the cutting clip 9 that is spread out is finely adjusted such that an appropriate part of the loop portion 41 can be gripped in a state where a joining portion 91 of the cutting clip 9 is gripped by the pair of arm portions 51a and 51a of the coupling hook 51 and the cutting clip 9 is connected to the distal end of the inner sheath 52. Next, the inner sheath 52 is pushed out to the distal end side with respect to the drive wire 53, a tightening ring 94 is slid to the distal end side, the pair of arm portions 92 and 92 of the cutting clip 9 are closed, and then an appropriate part of the loop portion 41 is put into a state of being gripped by the cutting clip 9 (clipped state) as illustrated in Fig. 10B. Subsequently, as illustrated in Fig. 10C, the outer sheath 54 is slid to the distal end side with respect to the inner sheath 52 and the distal end of the outer sheath 54 is allowed to abut against the loop portion 41. Next, the inner sheath 52 and the drive wire 53 are slid to the proximal end side with respect to the outer sheath 54 and the cutting clip 9 is pulled into the outer sheath 54. As a result, the appropriate part of the loop portion 41 is cut by the force that is generated by the distal end of the outer sheath 54 and the cutting clip 9.

In a case where the next cutting (cutting of the loop portion of another traction clip) is performed, the cutting clip 9 may be replaced with a new cutting clip. Accordingly, the problem of permanent set-in fatigue does not arise and cutting can be appropriately performed at all times, even when the cutting is repeated, as compared with a case where cutting is performed by means of the coupling hook 51 as illustrated in Figs. 8A to 8C.

The embodiment described above is to facilitate the understanding of the invention and does not limit the invention. Accordingly, each element disclosed in the embodiment described above is intended to include every change in design and equivalent belonging to the technical scope of the invention.

### EXPLANATIONS OF LETTERS OR NUMERALS

- 1: TRACTION CLIP FOR ENDOSCOPE
- 2: CLIP MAIN BODY
21 JOINING PLATE PORTION (JOINING PORTION)
22 ARM PLATE PORTION (ARM PORTION)
22a PROXIMAL END
22b GRIP PORTION
23 CLAW PORTION
24 TIGHTENING RING
25 CONNECTING HOLE
- 3: COIL SPRING (CONNECTING MEMBER)
31 MAIN BODY PORTION
32 CLIP HOOK
33 LOOP MEMBER HOOK
- 4: LOOP MEMBER
- 41: LOOP PORTION
- 42: ADAPTER PORTION
- 42a: SPRING THROUGH HOLE
- 41c: SMALL-DIAMETER PORTION (FRAGILE PORTION)
- 41d: LARGE-DIAMETER PORTION
- 41e: FRAGILE PORTION
- 5: CLIP DEVICE
- 51: COUPLING HOOK
- 52: INNER SHEATH
- 53: DRIVE WIRE
- 54: OUTER SHEATH
- 7: NORMAL CLIP
- 8: ELECTRIC KNIFE FOR AN ENDOSCOPE
- 9: CUTTING CLIP
- X: LESION SITE
- Y: FACING PART

## Claims

1. A traction clip for an endoscope comprising:
a clip main body including a pair of arm portions spreading out in a substantially V shape and a ring-shaped tightening ring externally fitted in a slidable manner to a joining portion on proximal end sides of the arm portions; and
a loop member made of a thermoplastic polymer attached to a part of the clip main body directly or via a connecting member, the loop member including a loop portion formed in a loop shape and having, at a part of the loop portion, a fragile portion cuttable with a force weaker than at the other part.

2. The traction clip for an endoscope according to claim 1, wherein a part of the loop portion serves as the fragile portion by being formed thinner than the other part.

3. A traction clip for an endoscope comprising:
a clip main body including a pair of arm portions spreading out in a substantially V shape toward distal end sides and a ring-shaped tightening ring externally fitted in a slidable manner to a joining portion on proximal end sides of the arm portions, the tightening ring being pushed and slid by a distal end of a sheath and closing the arm portions by an engagement member being pulled from the distal end of the sheath in a state where the engagement member is engaged with the joining portion by a clip device being used, the clip device having the sheath and the engagement member, and the engagement member being disposed so as to be capable of advancing from and retracting into the sheath and detachably engaging with the joining portion; and
a loop member made of a thermoplastic polymer attached to a part of the clip main body directly or via a connecting member, the loop member including a loop portion formed in a loop shape, the engagement member of the clip device being engageable with a part of the loop portion, and the loop member being configured to be cut by the engagement member being pulled from the distal end of the sheath in a state where the engagement member is engaged with a part of the loop portion by the clip device being used.

4. The traction clip for an endoscope according to claim 3, wherein the engagement member has a coupling hook spreading out in a substantially V shape by being pushed out from the distal end of the sheath and closing by being pulled into the sheath.

5. A traction clip for an endoscope comprising:
a clip main body including a pair of arm portions spreading out in a substantially V shape toward a distal end side and a ring-shaped tightening ring externally fitted in a slidable manner to a joining portion on proximal end sides of the arm portions, the tightening ring being pushed and slid by a distal end of an inner sheath and closing the arm portions by an engagement member being pulled from the distal end of the inner sheath in a state where the engagement member is engaged with the joining portion by a clip device being used and the clip device having the inner sheath, the engagement member disposed so as to be capable of advancing from and retracting into the inner sheath and detachably engaging with the joining portion, and an outer sheath through which the inner sheath is slidably inserted; and
a loop member made of a thermoplastic polymer attached to a part of the clip main body directly or via a connecting member, the loop member including a loop portion formed in a loop shape, the engagement member of the clip device being engageable with a part of the loop portion, and the loop member being configured to be cut by a cutting clip being pulled from a distal end of the outer sheath in a state where the engagement member is engaged with a joining portion of the cutting clip substantially similar configuration with the clip main body, the cutting clip is connected to the distal end of the inner sheath, and a part of the loop portion is gripped by a pair of arm portions of the cutting clip being closed, while using the clip device.

6. The traction clip for an endoscope according to claim 5, wherein the engagement member has a coupling hook spreading out in a substantially V shape by being pushed out from the distal end of the inner sheath and closing by being pulled into the inner sheath.
